# EUROPEAN PATENT APPLICATION

(11) **EP 4 570 312 A1**
(43) Date of publication of application: **18.06.2025**
(21) Application number: 24850224.7
(22) Date of filing: 08.03.2024
(51) Int. Cl.: A61N 7/00, A61N 1/40, A61N 1/06

(54) **COMBINED EXTRACORPOREAL SHOCK-WAVE AND RADIO-FREQUENCY TREATMENT DEVICE WITH ANTI-CELLULITE FUNCTION**

(30) Priority: 11.10.2023 KR 20230135351
(71) Applicant: K1med Global Inc., Seoul 08377 (KR)
(72) Inventor: KIM, Sangsik, Seoul 07034 (KR)
(74) Representative: dompatent von Kreisler Selting Werner - Partnerschaft von Patent- und Rechtsanwälten mbB
(86) International application number: PCT/KR2024/002985
(87) International publication number: WO 2025/079790

(57) **Abstract**

The disclosure relates to an extracorporeal shockwave and radio frequency (RF) combined treatment apparatus with an anti-cellulite function, including a housing provided in a tube shape having a predetermined inner installation space, and including an installation body coupled to a front end thereof, an extracorporeal shockwave therapy (ESWT) module installed in the housing to perform ESWT by accelerating a projectile using compressed air to collide with an applicator, and transmitting shockwaves generated upon the collision to a skin contacting a transmitter corresponding to a front end of the applicator through the transmitter, and an RF therapy module installed in the housing to perform RF therapy using a plurality of electrodes generating bipolar RF.

## Description

### [Technical Field]

The disclosure relates to an extracorporeal shockwave and radio frequency (RF) combined treatment apparatus with an anti-cellulite function.

### [Background Art]

Extracorporeal shockwave therapy (ESWT) is a sound wave therapy technique that increases pressure around a target organ using sound waves, followed by propagation of hypotonic sound waves. The biotherapeutic mechanism of ESWT studied to date is to induce hyperpolarization of the cell membrane of a target tissue and generate free radicals to induce healing.

ESWT is performed for the purpose of reducing pain and improving functions by applying shockwaves to a lesion from the outside of the body for medial epicondylitis, lateral epicondylitis of the humerus, plantar fasciitis, calcific tendonitis of a shoulder joint, and delayed fracture union to help revascularization and stimulate or reactivate a healing process of tendons and their surrounding tissues and bones.

Radio frequency (RF) therapy is a treatment method that uses a frequency of 0.3MHz to 5MHz to increase skin regeneration and elasticity. Regarding the mechanism of action, it is studied that an extracellular matrix existing in the subcutaneous fat layer is stimulated by RF transmitted through the dermis layer to increase skin regeneration and elasticity.

For RF therapy devices, there are a monopolar type which is used for massage, obesity treatment, and pain treatment by applying RF to a single plus electrode and allowing the RF to be refluxed from a ground electrode in contact with the skin, resulting in a wide field range and strong skin penetration, and a bipolar type which is used for local surgery or treatment by flowing RF energy to the skin from a plus electrode and allowing it to be refluxed to a ground electrode, resulting in a narrow electric field range and preventing tissue damage caused by stimulation outside a targeted range.

Each of these treatment methods should be performed individually through a single device specialized for the treatment method, and even when these methods are used in combination, treatment is performed only by sequentially using a plurality of devices, which causes a lot of hassle and inconvenience in a treatment process.

Accordingly, many technical attempts have been made to efficiently and effectively utilize ESWT and RF therapy. In this regard, there is a prior art document for providing an extracorporeal shockwave medical device with various functions, which is capable of selectively applying physical shock and electrical shock, Korean Patent Publication No. KR 10-2424812 entitled by "Extracorporeal shockwave medical device with high-frequency and low-frequency treatment functions" (hereinafter, referred to as "prior art").

However, technologies related to existing treatment devices for providing an ESWT function and an RF therapy function in combination, including the prior art, merely allow a user to selectively change the ESWT and RF therapy functions within one device and use them sequentially, and have limitations in simultaneously performing the functions in conjunction with each other in a composite manner.

### [Disclosure]

### [Technical Problem]

The disclosure is devised to address the above problems, and accordingly, an aspect of the disclosure is to provide a technique for simultaneously providing an extracorporeal shockwave therapy (ESWT) function and a radio frequency (RF) therapy function in conjunction with each other in a composite manner, and allowing synergy between the functions to be expected in performing the functions in conjunction with each other.

### [Technical Solution]

To achieve the object, an extracorporeal shockwave and radio frequency (RF) combined treatment apparatus with an anti-cellulite function includes a housing provided in a tube shape having a predetermined inner installation space, and including an installation body coupled to a front end thereof, an extracorporeal shockwave therapy (ESWT) module installed in the housing to perform ESWT by accelerating a projectile using compressed air to collide with an applicator, and transmitting shockwaves generated upon the collision to a skin contacting a transmitter corresponding to a front end of the applicator through the transmitter, and an RF therapy module installed in the housing to perform RF therapy using a plurality of electrodes generating bipolar RF. The installation body includes a first body part including a suction groove recessed rearward and having a cup shape, and having the transmitter installed to protrude from a center of a front surface thereof, and a second body part formed to extend from an outer side of the first body part to have a ring-shaped installation surface, and having the plurality of electrodes of the RF therapy module exposed forward and installed to be uniformly distributed radially around the transmitter at a center thereof.

The installation body may further include a suction hole providing part having a tube-shaped structure protruding rearward from at least one position of the first body part, and including a suction hole opened to communicate the suction groove with an inside of the housing therein, and the extracorporeal shockwave and RF combined treatment apparatus may further include a suction module connected to the suction hole providing part, and allowing a ring-shaped area between an area contacting the transmitter and an area contacting the plurality of electrodes of the RF therapy module, on the skin contacting the front end of the housing, to be sucked rearward and into the suction groove through suction, when the suction module is driven.

Further, the transmitter of the ESWT module may be installed with a front-back height of a front end thereof greater than a front-back height of front ends of the electrodes of the RF therapy module in a forward direction.

Further, the transmitter of the ESWT module may be installed with the front-back height of the front end thereof greater than the front-back height of the front ends of the electrodes of the RF therapy module in the forward direction by 6mm to 7mm.

Further, when the transmitter of the ESWT module has a diameter of 40mm to 50mm on a front surface thereof, the first body part may be provided in a form having a diameter of 70mm to 80mm on a front surface thereof.

The extracorporeal shockwave and RF combined treatment apparatus may further include a handle structure formed to extend from at least one position on a side surface of the housing to have a predetermined gripping space thereinside.

### [Advantageous Effects]

According to the disclosure, the following effects are achieved.

First, an extracorporeal shockwave therapy (ESWT) function and a radio frequency (RF) therapy function may be simultaneously provided in conjunction with each other in a composite manner, and synergy between the functions may also be expected in performing the functions in conjunction with each other.

Secondly, when ESWT is performed at a center of a skin contact surface of an apparatus and RF therapy is performed in an outer area thereof, the skin layer is pulled rearward through suction in an area between them to provide movement to the muscle layer located under the fat layer. Therefore, each therapy function may be performed more effectively.

Thirdly, the suction area makes a contact area of a transmitter denser, so that self-disintegration may be performed more effectively through blood circulation based on extracorporeal shockwaves. Therefore, a cellulite removal function may be further enhanced.

Fourthly, the level of effectiveness of a composite treatment function and synergy between the functions may be further enhanced by specifying an installation form of the transmitter of an ESWT module and an RF therapy module, and the formation structure of a suction groove.

### [Brief Description of the Drawings]

FIG. 1 is a perspective view illustrating the structure of an extracorporeal shockwave and radio frequency (RF) combined treatment apparatus according to the disclosure.
FIG. 2 is a front view illustrating the structure of the extracorporeal shockwave and RF combined treatment apparatus according to the disclosure.
FIG. 3 is a side view illustrating the structure of the extracorporeal shockwave and RF combined treatment apparatus according to the disclosure.
FIG. 4 is a cross-sectional view illustrating the installation structure of each module in the extracorporeal shockwave and RF combined treatment apparatus according to the disclosure.

### [Mode for Carrying Out the Invention]

A preferred embodiment of the disclosure will be described in more detail with reference to the attached drawings. However, a description of well-known technical parts will be omitted or briefly given, for brevity.

### <Description of extracorporeal shockwave and radio frequency (RF) combined treatment apparatus>

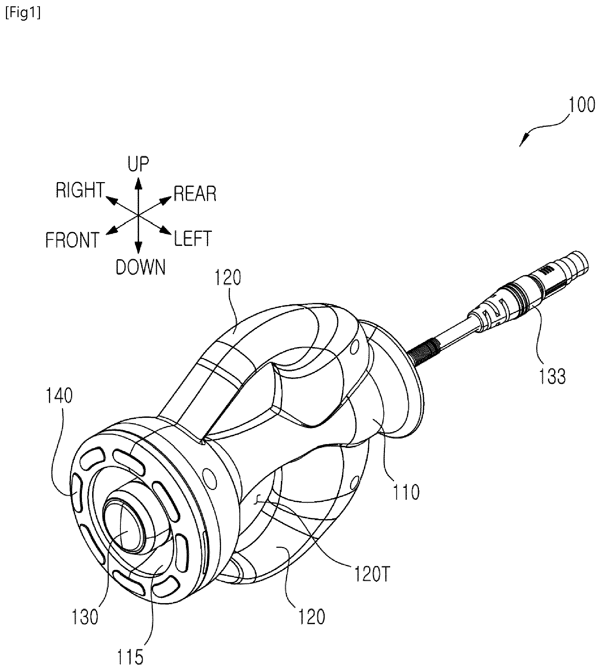

Referring to FIGS. 1 to 3, an extracorporeal shockwave and RF combined treatment apparatus 100 with an anti-cellulite function according to the disclosure is a device that may utilize an extracorporeal shockwave therapy (ESWT) function and a RF therapy function, selectively or in combination. For this purpose, the extracorporeal shockwave and RF combined treatment apparatus 100 includes a housing 110, a handle structure 120, an ESWT module 130, an RF therapy module 140, a suction module 150, and a control module (not shown).

The housing 110 corresponds to an overall outer body corresponding to a gun portion, which is provided in a tube shape with a predetermined inner installation space and directly held by an operator to perform treatment.

The housing 110 is provided, at a frond end thereof, with an installation body 115 coupled to specifically define the installation form of the ESWT module 130 and the RF therapy module 140, which will be described later.

Specifically, the installation body 115 includes a first body part 115a that determines the installation form of a front end of the ESWT module 130, a second body part 115b that determines the installation form of a front end of the RF therapy module 140, and a suction hole providing part 115c formed to be connected to the suction module 150.

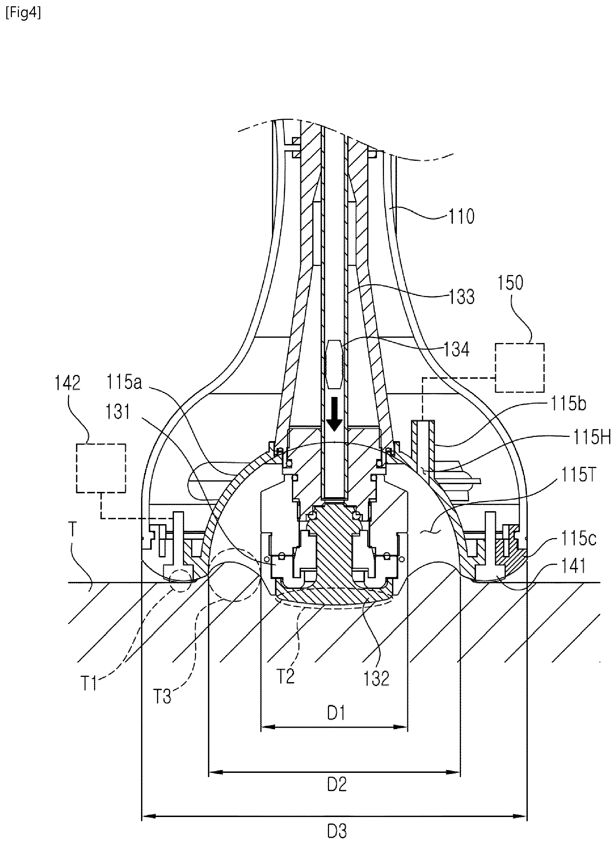

First, as illustrated in FIGS. 1 and 4, the first body part 115a is provided with a suction groove 115T which is recessed rearward and has a cup shape, and has a transmitter 132 installed to protrude from a center of a front surface of the first body part 115a.

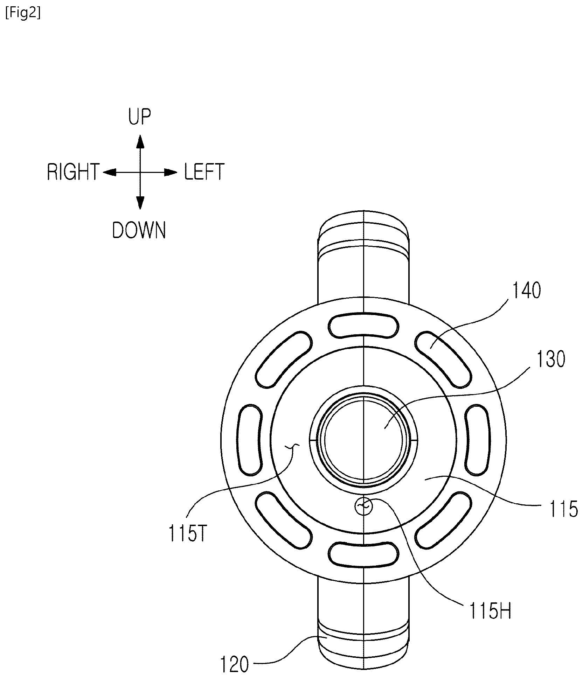

Further, the second body part 115b is formed to extend from an outer side of the first body part 115a to have a ring-shaped installation surface, so that a plurality of electrodes 141 of the RF therapy module 140 are installed to be exposed forward and uniformly distributed radially around the transmitter 132 at a center thereof, as illustrated in FIG. 2.

Most preferably, the plurality of electrodes 141 of the RF therapy module 140 are arranged to be equidistantly spaced apart from each other in a circle along the ring-shaped installation surface of the second body part 115b and thus to be radially distributed. As illustrated in FIG. 2, six or eight electrodes 141 are preferably arranged.

Finally, the suction hole providing part 115c has a tube-shaped structure protruding rearward from at least one position of the first body part 115a, and is provided with a suction hole 115H opened to communicate the suction groove 115T with the inside of the housing 110 therein.

A rear end of the suction hole providing part 115c is connected to a front end of a suction hose, and a rear end of the suction hose is connected to the suction module 150 to be described later.

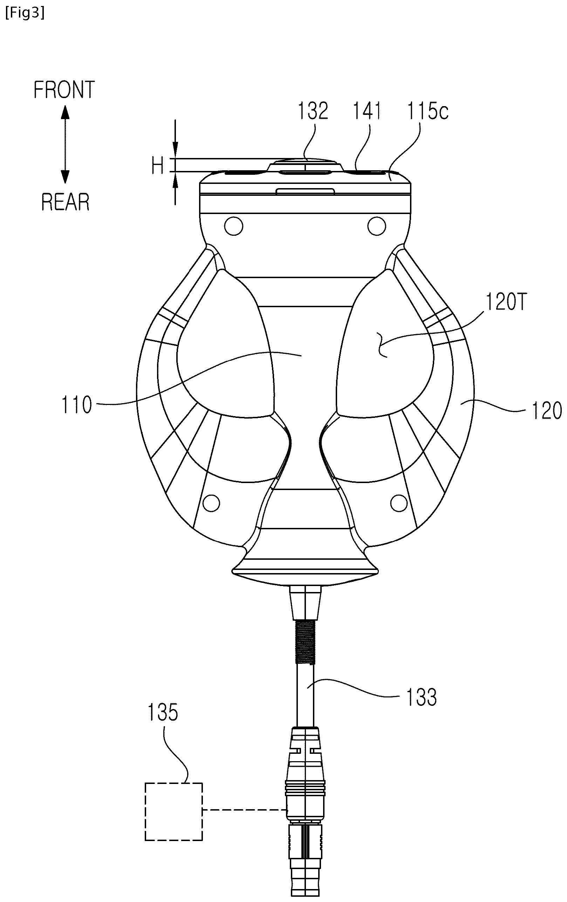

The handle structure 120 is a structural part extended from at least one position on a side of the housing 110 to have a predetermined gripping space 120T inside it. Most preferably, a pair of handle structures 120 are provided so that one of them is selectively gripped or both of them are gripped with both hands as illustrated in FIG. 3, to which the disclosure is not limited.

As illustrated in FIG. 4, the ESWT module 130 corresponds to a device that performs ESWT by accelerating a projectile 134 using compressed air and causing it to collide with an applicator 131, thereby transmitting shockwaves generated upon the collision to the skin contacting the transmitter 132 corresponding to the front end of the applicator 131.

Specifically, in the ESWT module 130 using air pressure to generate pressure waves, a compressed air output unit 135 accelerates the projectile 134 using compressed air based on an operation command from the control module (not shown), the projectile 134 moves toward a front end of a projectile moving tube 133 within the projectile moving tube 133 and collides with the applicator 131 connected to the front end of the projectile moving tube 133, and the applicator 131 transmits generated pressure waves to the body through the transmitter 132 corresponding to its front end, so that radial ESWT is performed within a specific center area.

In installing this ESWT module 130 in the housing 110, it is preferable that the projectile moving tube 133 and the projectile 134 are arranged in an internal space of the housing 110, and the applicator 131 and the transmitter 132 are exposed forward, with the front end of the transmitter 132 protruding outward from the front of the suction groove 115T provided in the first body part 115a.

Further, as illustrated in FIG. 3, the transmitter 132 of the ESWT module 130 is installed such that a front-back height of the front end of the transmitter 132 is greater than a front-back height of front ends of the electrodes of the RF therapy module by as much as H in a forward direction.

Therefore, the skin surface in an area T2 where ESTW is performed comes into closer contact, and at the same time, even the muscle layer under the fat layer in the skin is included in a suction area T3 sucked into the suction groove 115T in conjunction therewith, so that the muscle layer also experiences movement, thereby increasing the degree of blood circulation and enabling more effective removal of related cellulite.

Specifically, as illustrated in FIG. 3, the transmitter 132 of the ESWT module 130 is designed such that the difference H between the front-back height of the front end of the transmitter 132 which is protruded further forward and the front-back height of the front ends of the electrodes of the RF therapy module is within an appropriate range of 6mm to 7mm (most preferably, 6.72mm).

The RF therapy module 140 corresponds to a device that performs RF therapy using a plurality of electrodes generating bipolar RF.

Specifically, in the RF therapy module 140, an RF output unit 142 electrically connected to the electrodes 141 arranged radially around the transmitter 132 of the ESWT module 130 in the second body part 115b transmits an AC current in an RF range of 200kHz to 1200kHz, and frictional energy caused by vibration of ions in a tissue, which are generated in the process of the AC current spreading around on contact surfaces of the electrodes 141, increases the temperature of the tissue, thereby forming an RF therapy area T1 that induces coagulation necrosis.

To this end, six or eight electrodes 141 are provided in pairs so that when RF energy is applied to the skin from a plus electrode, it is refluxed to an adjacent ground electrode.

As a result, the transmitter 132 of the ESWT module 130 is formed to protrude from the center of a front surface of the installation body 115 of the housing 110, the suction groove 115T recessed in the shape of a cup in the first body part 115a is provided to surround the transmitter 132, and the plurality of electrodes 141 of the RF therapy module 140 are radially arranged on the installation surface of the second body part 115b surrounding an outer side of the groove structure.

In addition, when the transmitter 132 of the ESWT module 130 has an outermost diameter D1 of 40mm to 50mm on the front surface thereof, it is preferable that the first body part 115a is provided in a form having an outermost diameter D2 of 70mm to 80mm on the front surface thereof, and the second body part 115b is provided in a form having an outermost diameter D3 of 110mm to 120mm on the front surface thereof, as illustrated in FIG. 4.

Most preferably, the outermost diameter D1 of the transmitter 132 of the ESWT module 130 on the front surface thereof is 43mm to 45mm, the outermost diameter D2 of the first body part 115a on the front surface thereof is 75mm to 76mm, and the outermost diameter D3 of the second body part 115b on the front surface thereof is 115mm to 116mm.

Therefore, the skin surface in the area T2 where ESTW is performed comes into closer contact, and at the same time, even the muscle layer under the fat layer in the skin is included in the suction area T3 sucked into the suction groove 115T in conjunction therewith, so that the muscle layer also experiences movement, thereby increasing the degree of fat melting and enabling more effective targeting of a functional area.

The suction module 150 is connected to the suction hole providing part 115c, so that when the suction module 150 is driven, the ring-shaped area T1 between the area T2 in contact with the transmitter 132 and the area T2 in contact with the plurality of electrodes of the RF therapy, on the skin contacting the front end of the housing 110, is sucked rearward and into the suction groove 115T through suction.

Specifically, the suction module 150 allows the skin to be sucked into the suction groove 115T through suction so that an area from the epidermis layer through the dermis layer, the fascia layer, and the fat layer to the muscle layer is lifted, and movement is imparted to the muscle layer as well.

The control module (not shown) is electrically connected to the compressed air output unit 135 of the ESWT module 130, the RF output unit 142 of the RF therapy module 140, and the suction module 150 to control an operation state of each component. That is, as the operation states of the compressed air output unit 135 of the ESWT module 130, the RF output unit 142 of the RF therapy module 140, and the suction module 150 are controlled by a control command generated based on a signal input to the control module, the functions of ESWT, RF therapy, and suction may all be performed simultaneously or selectively.

A test was conducted to verify an actual clinical effect using the extracorporeal shockwave and RF combined treatment apparatus 100 with the anti-cellulite function according to the disclosure described above. The results of Table 1 below were obtained by observing skin conditions and temperature changes before and after the functions of ESWT, RF therapy, and suction were all performed at once.

**[Table 1]**

| | Before treatment | After treatment |
|---|---|---|
| Abdomen | 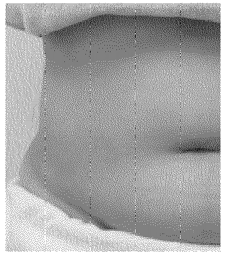 | 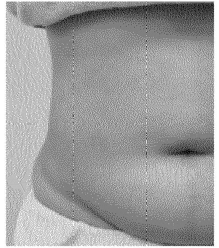 |
| Abdomen (thermal imaging camera) | 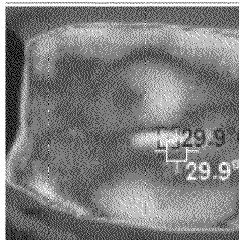 | 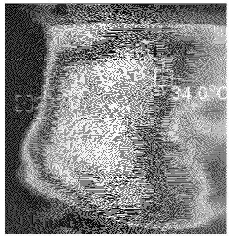 |
| Leg | 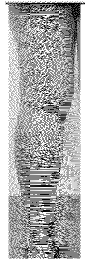 | 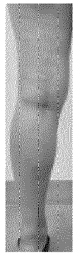 |
| Leg (thermal imaging camera) | 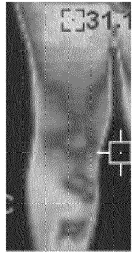 | 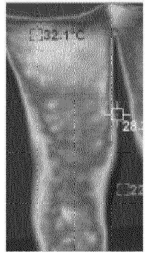 |

As illustrated in Table 1, when the extracorporeal shockwave and RF combined treatment apparatus 100 with the anti-cellulite function according to the disclosure is used, the temperature of the abdomen increased from about 29°C to about 34°C, with a temperature change of +5°C to +6°C, and the temperature of the leg increased from about 31°C to about 32°C, with a temperature change of +1°C to +2°C.

This is a significant functional improvement, considering that the abdomen showed a +3°C temperature change with a temperature increase from about 30°C to about 33°C, and the leg showed a +1°C temperature change with a temperature increase within about 31°C, as verified in the same test with a conventional device that performs RF therapy alone.

In fact, as seen in the photographs of the abdomen and leg, the degree of blood circulation and cellulite removal are also very good.

Therefore, it may be seen that in the configuration of the extracorporeal shockwave and RF combined treatment apparatus 100 with the anti-cellulite function according to the disclosure, the installation and operation configurations of the ESWT module 130, the RF therapy module 140, and the suction module 150 enhance the levels of ESWT and RF therapy functions and significantly improve synergy between them, in simultaneously providing the ESWT and RF therapy functions in conjunction with each other in a composite manner.

The embodiment of the disclosure is intended to describe the technical ideas of the disclosure, not to limit them, and the scope of the disclosure not limited by the embodiment. The scope of protection is to be construed in accordance with the appended claims, and all technical ideas within the scope of the equivalents thereof are to be construed to be encompassed in the scope of the disclosure.

### [Description of reference numerals]

| | | | |
|---|---|---|---|
| 100: | Extracorporeal shockwave and RF treatment apparatus | | |
| 110: | Housing | | |
| 115: | Installation body | | |
| 115a: | First body part | 115b: | Second body part |
| 115T: | Suction hole | 115c: | Suction hole providing part |
| 115H: | Suction hole | | |
| 120: | Handle structure | | |
| 120T: | Gripping space | | |
| 130: | ESWT module | | |
| 131: | Applicator | 132: | Transmitter |
| 133: | Projectile moving tube | 134: | Projectile |
| 135: | Compressed air output unit | | |
| 140: | RF therapy module | | |
| 141: | Electrode | 142: | RF output unit |
| 150: | Suction module | | |

## Claims

1. An extracorporeal shockwave and radio frequency (RF) combined treatment apparatus with an anti-cellulite function, comprising:
a housing provided in a tube shape having a predetermined inner installation space, and including an installation body coupled to a front end thereof;
an extracorporeal shockwave therapy (ESWT) module installed in the housing to perform ESWT by accelerating a projectile using compressed air to collide with an applicator, and transmitting shockwaves generated upon the collision to a skin contacting a transmitter corresponding to a front end of the applicator through the transmitter; and
an RF therapy module installed in the housing to perform RF therapy using a plurality of electrodes generating bipolar RF,
wherein the installation body includes:
a first body part including a suction groove recessed rearward and having a cup shape, and having the transmitter installed to protrude from a center of a front surface thereof; and
a second body part formed to extend from an outer side of the first body part to have a ring-shaped installation surface, and having the plurality of electrodes of the RF therapy module exposed forward and installed to be uniformly distributed radially around the transmitter at a center thereof.

2. The extracorporeal shockwave and RF combined treatment apparatus of claim 1, wherein the installation body further includes a suction hole providing part having a tube-shaped structure protruding rearward from at least one position of the first body part, and including a suction hole opened to communicate the suction groove with an inside of the housing therein, and
wherein the extracorporeal shockwave and RF combined treatment apparatus further comprises a suction module connected to the suction hole providing part, and allowing a ring-shaped area between an area contacting the transmitter and an area contacting the plurality of electrodes of the RF therapy module, on the skin contacting the front end of the housing, to be sucked rearward and into the suction groove through suction, when the suction module is driven.

3. The extracorporeal shockwave and RF combined treatment apparatus of claim 2, wherein the transmitter of the ESWT module is installed with a front-back height of a front end thereof greater than a front-back height of front ends of the electrodes of the RF therapy module in a forward direction.

4. The extracorporeal shockwave and RF combined treatment apparatus of claim 3, wherein the transmitter of the ESWT module is installed with the front-back height of the front end thereof greater than the front-back height of the front ends of the electrodes of the RF therapy module in the forward direction by 6mm to 7mm.

5. The extracorporeal shockwave and RF combined treatment apparatus of claim 3, wherein when the transmitter of the ESWT module has a diameter of 40mm to 50mm on a front surface thereof, the first body part is provided in a form having a diameter of 70mm to 80mm on a front surface thereof.

6. The extracorporeal shockwave and RF combined treatment apparatus of claim 1, further comprising a handle structure formed to extend from at least one position on a side surface of the housing to have a predetermined gripping space thereinside.
